# EUROPEAN PATENT SPECIFICATION

(11) **EP 2 141 497 B1**
(45) Date of publication and mention of the grant of the patent: **26.03.2014**
(21) Application number: 09163976.5
(22) Date of filing: 29.06.2009
(51) Int. Cl.: G01N 33/558, B01L 3/00

(54) **Method for the analysis of circulating antibodies**
Verfahren zur Analyse von umlaufenden Antikörpern
Procédé pour l'analyse d'anticorps circulants

(30) Priority: 03.07.2008 SE 0801587; 03.07.2008 US 78295 P
(43) Date of publication of application: 06.01.2010
(73) Proprietor: Amic AB, 751 83 Uppsala (SE)
(72) Inventor: Mendel-Hartvig, Ib, 756 55, Uppsala (SE); Pettersson, Christer, 743 35, Storvreta (SE); Rundström, Gerd, 752 41, Uppsala (SE)
(74) Representative: Carlsson, Carl Fredrik Munk

(56) References cited:
- WO-A-03/103835
- WO-A-2004/029221
- WO-A-2005/118139
- WO-A-2007/149043
- US-A- 4 275 053
- US-A- 5 192 663
- US-A- 5 773 222

## Description

### Technical field

The present invention concerns a method for the analysis circulating antibodies.

### Background

Quick, reliable, and cost effective analytical and diagnostic methods are desirable.

WO03/103835 (Åmic AB) relates to a micro fluidic system comprising a substrate and provided on said substrate there is at least one flow path comprising a plurality of micro posts protruding upwards from said substrate, the spacing between the micro posts being small enough to induce a capillary action in a liquid sample applied, so as to force said liquid to move. There is disclosed that the device can comprise a denser zone which can act as a sieve preventing for instance cells to pass. There is also disclosed an embodiment with microstructures where the shape, size and/or center-to-center distance forms a gradient so that cells and the like can be delayed or separated.

WO2005/089082 (Åmic AB) shows a device and method for the separation of a component in a liquid sample prior to the detection of an analyte in said sample, wherein a sample is added to a receiving zone on a substrate, said substrate further optionally comprising a reaction zone, a transport or incubation zone connecting the receiving and reaction zone, respectively, forming a flow path on a substrate, wherein said substrate is a non-porous substrate, and at least part of said flow path consists of areas of projections substantially vertical to the surface of said substrate, and having a height, diameter and reciprocal spacing such, that lateral capillary flow of said liquid sample in said zone is achieved, and where means for separation are provided adjacent to the zone for receiving the sample. There is disclosed an embodiment where red blood cells are removed.

WO2005/118139 (Åmic AB) concerns a device for handling liquid samples, comprising a flow path with at least one zone for receiving the sample, and a transport or incubation zone, said zones connected by or comprising a zone having projections substantially vertical to its surface, said device provided with a sink with a capacity of receiving said liquid sample, said sink comprising a zone having projections substantially vertical to its surface, and said sink being adapted to respond to an external influence regulating its capacity to receive said liquid sample. It is disclosed that the device can be used when particulate matter such as cells is to be removed from the bulk of the sample. It is stated that red blood cells can be separated without significant rupture of the cells.

WO2004/029221 (The General Hospital Corporation, and GPB Scientific LLC) discloses methods for separating cells from a sample. There is disclosed the separation of cells with different properties. The devices are closed devices with an input and output channel and a lid. The device comprises arrays of obstacles that are capable of binding a population of cells.

US 2007/0059718 A1 discloses methods for detecting and concentrating biohazard analytes such as bacteria, protozoa, viral pathogens, and toxins.

There is a need for a robust and reliable method for the analysis of circulating antibodies.

### Summary of the invention

One object of the present invention is to provide an improved method for the analysis of circulating antibodies.

There is provided a method for the analysis of circulating antibodies in a liquid sample, said method comprising the steps:
a. providing an analysis device comprising a substrate, and provided on said substrate at least one sample addition zone, at least one retaining zone, at least one sink, and at least one flow path connecting the sample addition zone, the retaining zone and the sink, wherein the flow path is open and comprises projections substantially vertical to the surface of said substrate and having a height (H), diameter (D) and reciprocal spacing (t1, t2) such that lateral capillary flow of said liquid sample is achieved and such that cells can flow through the projections, wherein said retaining zone comprises at least one affinity binding means to which cell structures are bound,
b. adding at least one sample to a sample addition zone, and
c. reading a result,
wherein circulating antibodies directed against bound cell structures are determined.

Further aspects and embodiments of the present invention are defined in the appended claims which are incorporated herein by reference.

By providing a substrate comprising projections in combination with a retaining zone where particles and/or cells are retained by attractive forces, several advantages are obtained.

The projections give a large surface for the substrate and the large surface of the retaining zone is an advantage because cells are bound more efficiently. The kinetics of the analysis device is improved with the combination of projections and affinity binding.

The projections in combination with the affinity binding mean provide a possibility to create a suitable flow of sample liquid in the device. This allows problems such as unwanted clogging to be avoided.

A further advantage of the present invention is that the reading of a result is easier in an open system according to the present invention. Moreover there are no problems with entrapped gases in an open system.

Another advantage of using substantially vertical projections to analyse cells is that this allows design of the projections so that the cells are handled carefully.

### Definitions

Before the present device and method is described, it is to be understood that this invention is not limited to the particular configurations, method steps, and materials disclosed herein as such configurations, steps and materials may vary somewhat. It is also to be understood that the terminology employed herein is used for the purpose of describing particular embodiments only and is not intended to be limiting since the scope of the present invention will be limited only by the appended claims. It must also be noted that, as used in this specification and the appended claims, the singular forms "a", "an", and "the" include plural referents unless the context clearly dictates otherwise. Thus, for example, reference to a reaction mixture containing "an antibody" includes a mixture of two or more antibodies.

The term "about" when used in the context of numeric values denotes an interval of accuracy, familiar and acceptable to a person skilled in the art. Said interval is ± 10 %.

In describing and claiming the device and method, the following terminology will be used in accordance with the definitions set out herein.

As used throughout the claims and the description the wording "affinity binding means to which cells are bound" denotes an element which binds to cells by attractive forces between the binding means and the cells.

As used throughout the claims and the description the term "analysis" means the process in which at least one analyte is determined.

As used throughout the claims and the description the term "analysis device" means a device by the aid of which an analysis can be performed.

As used throughout the claims and the description the term "analyte" means a substance or chemical or biological constituent of which one or more properties are determined in an analytical procedure. An analyte or a component itself can often not not be measured, but a measurable property of the analyte can. For instance, it is possible to measure the concentration of an analyte.

As used throughout the claims and the description the term "capillary flow" means flow induced mainly by capillary force.

As used throughout the claims and the description the term "casing" means an element enclosing a part of or the entire device.

As used throughout the claims and the description the term "circulating antibody" means an antibody in solution.

As used throughout the claims and the description the term "detectable group" means any arrangement of molecules or atoms that can be detected when present on a substrate.

As used throughout the claims and the description the term "flow path" means an area on the device where flow of liquid can occur between different zones.

As used throughout the claims and the description the term "fluid connection" means a connection in which a fluid can be transported.

As used throughout the claims and the description the term "lid" means an element covering a part of or the entire device.

As used throughout the claims and the description the term "open" used in connection with capillary flow means that the system is open i.e. the system is not enclosed. Examples of an open system include a system without at lid in capillary contact with the sample liquid. In an open system a lid shall not be in capillary contact with the sample liquid, i.e. a lid shall not take part in creating the capillary force.

As used throughout the claims and the description the term "reciprocal spacing" means the distance between adjacent projections.

As used throughout the claims and the description the term "retaining zone" means a zone where at least some part of a sample is retained.

As used throughout the claims and the description the term "sample" means a mixture or a solution to be analysed.

As used throughout the claims and the description the term "sample addition zone" means a zone where a sample is added.

As used throughout the claims and the description the term "sink" means an area with the capacity of receiving liquid sample.

As used throughout the claims and the description the term "substance" means any pure chemical or biological entity or any mixture or solution comprising at least one chemical or biological entity.

### Detailed description

In a first aspect there is provided a method for the analysis of circulating antibodies in a liquid sample, said method comprising the steps:
a) providing an analysis device comprising a substrate, and provided on said substrate at least one sample addition zone, at least one retaining zone, at least one sink, and at least one flow path connecting the sample addition zone, the retaining zone and the sink, wherein the flow path is open and comprises projections substantially vertical to the surface of said substrate and having a height (H), diameter (D) and reciprocal spacing (t1, t2) such that lateral capillary flow of said liquid sample is achieved and such that cells can flow through the projections, wherein said retaining zone comprises at least one affinity binding means to which cell structures are bound,
b) adding at least one sample to a sample addition zone, and
c) reading a result,
wherein circulating antibodies directed against bound cell structures are determined.

Reciprocal spacing (t1, t2) denotes the reciprocal spacing in x and y direction in an orthogonal coordinate system. In one embodiment all projections have the same spacing in x-direction and/or y-direction. In an alternative embodiment the projections have different spacings in the x-direction. In one embodiment the distance of the different projections in x-direction are t1₁, t1₂, t1₃... In a further embodiment the projections have different spacings in the y-direction. In one embodiment the distance of the different projections in y-direction are t2₁, t2₂, t2₃...

The device comprises a substrate. In one embodiment the substrate is partly or entirely enclosed by a casing or a lid. If a casing or a lid is used, the distance between the substrate is such that the casing or lid does not contribute to the capillary force acting on the sample liquid.

There is at least one sample addition zone to which sample liquid is added. There is a flow path in fluid connection with the sample addition zone and the retaining zone and the sink.

In one embodiment the sample flows in a flow path from a sample addition zone via a retaining zone to a sink.

In one embodiment the retaining zone is placed across the entire path/paths where the sample fluid flows so that no sample liquid is able to pass by the retaining zone. In an alternative embodiment the retaining zone is placed so that a part of the sample liquid passes the retaining zone without any essential interaction with the retaining zone.

In one embodiment at least one of a) the sample addition zone, b) the retaining zone and c) the sink, comprises projections substantially vertical to the surface of said substrate and having a height (H), diameter (D) and reciprocal spacing (t1, t2) such that lateral capillary flow of said sample is achieved and such that cells can flow through the projections.

In one embodiment the height, diameter and reciprocal spacing of the a) flow path, b) the sample addition zone, c) the retaining zone, and d) the sink are the same. In an alternative embodiment the height, diameter and reciprocal spacing of at least one of a) flow path, b) the sample addition zone, c) the retaining zone, and d) the sink are different.

In one embodiment the affinity binding means is selected from an antibody, an aptamer, a receptor, a ligand, a single chain antibody, a fragmented antibody, and a lectin.

In one embodiment the micro posts are arranged with micro post distances of 5-200 µm. In another embodiment the micro post distances is 20-100 µm.

In one embodiment the micro posts are arranged with micro post heights of 1-1000 µm. In another embodiment the micro posts height is 10-100 µm.

In one embodiment the liquid sample is selected from the group consisting of human or animal blood, urine, lung liquids, synovial fluid, wound liquids, saliva, tears, and sweat.

In one embodiment the liquid sample is from human blood.

In one embodiment the cell structures are part of the haematological antigen system.

In one embodiment the cell structures are part of the antigens involved in HIV infection or detection.

In one embodiment the liquid sample is from human blood and is used for the determination of circulating antibodies directed against bacteria, viruses or small sized single or multi cell infectious agents.

In one embodiment the liquid sample is from human bone marrow.

Other features and uses of the invention and their associated advantages will be evident to a person skilled in the art upon reading the description and the examples.

It is to be understood that this invention is not limited to the particular embodiments shown here. The following examples are provided for illustrative purposes and are not intended to limit the scope of the invention since the scope of the present invention is limited only by the appended claims.

### Examples

### Example 1

### Adherence of cells to the an analyse device according to the invention.

The projections of the chip had different centre to centre spacing with the largest spacing in the flow direction. The projections were narrowing towards the top. The height of the projections was 65 µm. The diameter of the projections at the bottom was 70 µm and the diameter at the top was 50 µm. The spacing between the projections were t1 = t2 = 31.77 µm at the bottom of the projections and t1=t2= 51.77 µm at the top of the projections.

The principle of adherence of cells to the device surface is exemplified by firm binding of red blood cells (RBC). RBCs was firmly attached during free flow to a defined area of the chip surface by means of different principles including RBC agglutinins, charge and antibodies directed to surface antigens.

Small amounts (0.1 µl) of lectins 1 mg/ml in 50 mM Naphosphate buffer, pH 7.5 were applied in a single lane on the chip where after 20 µl RBCs 0.8 % in suspension were applied and let to flow through the detection zone containing the lectins. The results showed a variable RBC binding to different lectins (PHA-E, PHA-M, WGA, Jacalin) with WGA as the most efficient one. The bound RBCs that are clearly visible by eye remained attached after washing with 50 µl of buffers containing e.g. 0.1 % of the detergent Tween 20. Bound RBCs was determined quantitatively by adding 10 µl of rabbit anti-human RBC in combination with 10 µl of Cy5 goat anti-rabbit IgG.
A firm binding of RBC to the chip surface was also obtained using antibodies against RBC surface antigens such as glycophorin, a major surface protein of the human RBC.

Polylysine of high molecular weight which generally bind cells firmly in cell cultures was also able to bind RBC in numbers comparable to WGA.

Attachment of RBC to the 4castchip was also possible using biotin labelled RBC in combination with deposited streptavidin. Streptavidin (0.13 µl of 2 mg streptavidin/ml) in PBS pH 7,5 was applied in a single lane on the chip. 20 µl of RBCs 1.6 % labelled with Biotin using Sulfo-NHS-biotin was let to flow through the detections zone containing the streptavidin. The results showed a clearly visible firm binding of RBC to the streptavidin and remained attached after washing with 80 µl of buffers containing e.g. 0.1 % of the detergent Tween 20.

### Example 2

### Detection of soluble human antibodies directed to RBC surface antigens (indirect antiglobin test, IAT).

The principle of antibody detection is exemplified with detection of anti-D antibodies present in low titre in human serum. The assay principle involves firm adherence or binding of viable RBCs on the chip surface by means of deposited catching e.g. antibodies against RBC surface antigens. The same device as in example 1 was used. Thus, RBCs that are transported by free flow through the micro pillars of the chip are captured by chip bound antibodies located in the detection zone. A small volume (10 µl)of a human serum samples diluted 1:100 in LISS buffer containing 0.5 % gelatine containing anti-D antibodies of different titres was applied to sensitize the RBCs. Following washing (30 µl) the presence of IgG on the RBC surface was detected using 10 µl of an anti-human globulin antibody (AHG) conjugated with transfluosphere. The results showed a dose dependent binding of AHG conjugate to RBCs with respect to the anti-D antibody titre. Optimal sensitization was obtained in the absence of detergent and using low -ionic- strength saline (LISS) washing buffer containing 0,5% gelatine.

In the high sensitivity IAT assay the detection with the AHG-conjugate is done with fluorescent dye combinations with extremely large stoke shift (the separation between excitation and emission maxima) like in the Transfluosphere and europium conjugates.

### Example 3

### ABO blood group antigen testing

The ABO blood group antigens on RBCs are determined with high specificity. RBCs from donor blood samples were prepared by washing twice in LISS buffer and then re-suspended to a 0.8% RBC solution. The washed donor RBCs (4 %, 20 µl) in LISS buffer were attached to the device using deposited anti-glycophorin (1 mg/ml, 0.13 µl/chip). The A- and B-antigens respectively were detected using 10 µl of monoclonal anti-RBC-A and RBC-B antibodies followed by 10 µl of an anti-mouse IgM antibody conjugated with Cy5. The chip was finally washed with 60 µl of an assaybuffer (20 mM Tris, 0.135 M NaCl, 10 mM EDTA, 0.1% Tween 20, 1% BSA, pH 7.4).The fluorescence signal read at 635 nm was clearly positive for A positive RBCs and negative (equal to background signal) for B positive RBCs using anti- RBC-A antibodies. The same high specificity was obtained in experiments with B positive RBCs.

## Claims

1. A method for the analysis of circulating antibodies in a liquid sample, said method comprising the steps:
a. providing an analysis device comprising a substrate, and provided on said substrate at least one sample addition zone, at least one retaining zone, at least one sink, and at least one flow path connecting the sample addition zone, the retaining zone and the sink, wherein the flow path is open and comprises projections substantially vertical to the surface of said substrate and having a height (H), diameter (D) and reciprocal spacing (t1, t2) such that lateral capillary flow of said liquid sample is achieved and such that cells can flow through the projections, wherein said retaining zone comprises at least one affinity binding means to which cell structures are bound,
b. adding at least one liquid sample to a sample addition zone, and
c. reading a result,
wherein circulating antibodies directed against bound cell structures are determined.

2. The method according to claim 1, wherein said liquid sample is selected from the group consisting of human or animal blood, urine, lung liquids, synovial fluid, wound liquids, saliva, tears, and sweat.

3. The method according to any one of claims 1-2, wherein said liquid sample is from human blood.

4. The method according to any one of claims 1-3, wherein said cell structures are part of the haematological antigen system.

5. The method according to any one of claims 1-4, wherein said cell structures are part of the antigens involved in HIV infection or detection.

6. The method according to any one of claims 1-5, wherein said liquid sample is from human blood and is used for the determination of circulating antibodies directed against bacteria, viruses or small sized single or multi cell infectious agents.

7. The method according to any one of claims 1-6, wherein said liquid sample is from human bone marrow.

## Patentansprüche

1. Verfahren zur Analyse von zirkulierenden Antikörpern in einer flüssigen Probe, wobei das Verfahren folgende Schritte umfasst:
a. Bereitstellen einer Analysevorrichtung, die ein Substrat umfasst, und auf dem Substrat angeordnet mindestens einen Probenzugabebereich, mindestens einen Haltebereich, mindestens eine Vertiefung und mindestens einen Strömungsweg, der den Probenzugabebereich, den Haltebereich und die Vertiefung verbindet, wobei der Strömungsweg offen ist und Fortsätze im Wesentlichen senkrecht zur Oberfläche des Substrats umfasst, die eine Höhe (H), einen Durchmesser (D) und einen beiderseitigen Abstand (t1, t2) aufweisen, sodass eine seitliche Kapillarströmung der flüssigen Probe erreicht wird und sodass Zellen durch die Fortsätze strömen können, wobei der Haltebereich mindestens ein Affinitätsbindungsmittel umfasst, an das Zellstrukturen gebunden sind,
b. Zugeben mindestens einer flüssigen Probe in einen Probenzugabebereich, und
c. Ablesen eines Ergebnisses,
wobei zirkulierende Antikörper, die gegen gebundene Zellstrukturen gerichtet sind, bestimmt werden.

2. Verfahren nach Anspruch 1, wobei die flüssige Probe aus der Gruppe ausgewählt wird, die aus menschlichem oder tierischem Blut, Urin, Lungenflüssigkeiten, Synovialflüssigkeit, Wundflüssigkeiten, Speichel, Tränen und Schweiß besteht.

3. Verfahren nach Anspruch 1 oder 2, wobei die flüssige Probe aus menschlichem Blut stammt.

4. Verfahren nach einem der Ansprüche 1 bis 3, wobei die Zellstrukturen Bestandteil des Antigensystems im Blut sind.

5. Verfahren nach einem der Ansprüche 1 bis 4, wobei die Zellstrukturen Bestandteil der Antigene sind, die an einer HIV-Infektion oder an deren Nachweis beteiligt sind.

6. Verfahren nach einem der Ansprüche 1 bis 5, wobei die flüssige Probe aus menschlichem Blut stammt und zum Bestimmen von zirkulierenden Antikörpern verwendet wird, die gegen Bakterien, Viren oder kleine einzellige oder mehrzellige Krankheitserreger gerichtet sind.

7. Verfahren nach einem der Ansprüche 1 bis 6, wobei die flüssige Probe aus menschlichem Knochenmark stammt.

## Revendications

1. Procédé pour l'analyse d'anticorps circulants dans un échantillon de liquide, ledit procédé comprenant les étapes consistant à :
a. mettre à disposition un dispositif d'analyse comprenant un substrat, et sur ledit substrat au moins une zone d'addition d'échantillon, au moins une zone de retenue, au moins un puits et au moins une voie d'écoulement reliant la zone d'addition d'échantillon, la zone de retenue et le puits, la voie d'écoulement étant ouverte et comprenant des saillies essentiellement verticales par rapport à la surface dudit substrat et présentant une hauteur (H), un diamètre (D) et un espacement entre elles (t1, t2) tels que l'écoulement capillaire latéral dudit échantillon de liquide est obtenu et tels que des cellules peuvent circuler à travers les saillies, ladite zone de retenue comprenant au moins un moyen de liaison par affinité auquel des structures cellulaires sont liées,
b. ajouter au moins un échantillon de liquide sur une zone d'addition d'échantillon, et
c. lire un résultat,
dans lequel des anticorps circulants dirigés contre des structures cellulaires liées sont déterminés.

2. Procédé selon la revendication 1, dans lequel ledit échantillon de liquide est sélectionné dans le groupe constitué du sang humain ou animal, de l'urine, des liquides pulmonaires, du liquide synovial, des fluides de plaie, de la salive, des larmes et de la sueur.

3. Procédé selon l'une quelconque des revendications 1 à 2, dans lequel ledit échantillon de liquide provient de sang humain.

4. Procédé selon l'une quelconque des revendications 1 à 3, dans lequel lesdites structures cellulaires font partie du système d'antigènes du sang.

5. Procédé selon l'une quelconque des revendications 1 à 4, dans lequel lesdites structures cellulaires font partie des antigènes impliqués dans une infection par le VIH ou dans sa détection.

6. Procédé selon l'une quelconque des revendications 1 à 5, dans lequel ledit échantillon de liquide provient de sang humain et est utilisé pour la détermination d'anticorps circulants dirigés contre des bactéries, des virus ou des agents infectieux unicellulaires ou pluricellulaires de petite taille.

7. Procédé selon l'une quelconque des revendications 1 à 6, dans lequel ledit échantillon de liquide provient de moelle osseuse humaine.
